# EUROPEAN PATENT APPLICATION

(11) **EP 4 834 405 A1**
(43) Date of publication of application: **04.11.2026**
(21) Application number: 26170082.7
(22) Date of filing: 31.03.2026
(51) Int. Cl.: A61M 25/00, A61M 25/06

(54) **ANTI-BENDING INTRACRANIAL DOUBLE-TUBE GUIDE CATHETER**

(30) Priority: 30.04.2025 CN 202510559252
(71) Applicant: Sichuan Medact Technology Co., Ltd, Chengdu, Sichuan 610200 (CN)
(72) Inventor: HUANG, Fengkang, Chengdu, Sichuan 610200 (CN); YUAN, Wenwen, Chengdu City, Sichuan Province 610200 (CN); YIN, Lei, Chengdu, Sichuan 610200 (CN); CHEN, Dong, Chengdu, Sichuan 610200 (CN)
(74) Representative: Cleanthous, Marinos

(57) **Abstract**

Disclosed is an anti-bending intracranial double-tube guide catheter, including a catheter body formed by an outer catheter and an inner catheter in the outer catheter; at least two anti-bending sections are arranged along the catheter body, with an anti-bending support mechanism provided in each of the anti-bending sections; the anti-bending support mechanism includes an annular cavity (21) in the outer catheter and memory alloy spiral wires (22) circumferentially arranged in the annular cavity, with temperature control tubes (23) provided in intervals therebetween; each of the temperature control tubes is S-shaped in the annular cavity, and is connected to the outside by means of a circulation pipe penetrating the outer catheter; a temperature medium is injected into each of the temperature control tubes by means of the circulation pipe, to activate the memory alloy spiral wires, so that a shape memory effect occurs, to dynamically adjust the bending stiffness of the catheter.

## Description

### RELATED APPLICATIONS

The present application claims the benefit of priority of Chinese Patent Application No. 2025105592526, filed April 30, 2025, and entitled "ANTI-BENDING INTRACRANIAL DOUBLE-TUBE GUIDE CATHETER" the entire content of which is incorporated herein by reference.

### TECHNICAL FIELD

The present application relates to the technical field of medical catheters, and specifically to an anti-bending intracranial double-tube guide catheter.

### BACKGROUND

In neurointerventional surgery, the performance of the intracranial guide catheter has a significant impact on the surgical effect. Therefore, the anti-bending performance and control stability of the intracranial guide catheter will directly affect the safety of the operation. The intracranial guide catheter needs to have excellent anti-bending performance and controllable stiffness to cope with the complex three-dimensional tortuous anatomical structure of intracranial vessels. Currently, double-lumen guide catheters used in clinical practice are mostly made of braided layers or polymer composite layer structures, and their stiffness characteristics are frozen in the design stage, making it difficult for them to adapt to the complex and tortuous shapes of intracranial vessels. Even though the mechanical properties of catheters in the prior art have been improved by using composite materials and structural optimization (including applying a gradient stiffness design from the proximal end to the distal end), the following key defects remain:

the stiffness characteristics of catheters in the prior art are frozen in the manufacturing stage, so that the characteristics, such as the elastic modulus of the metal braided layer or polymer material, cannot be adjusted in real time with the change of the vascular morphology during surgery. Some catheters are designed with decreasing stiffness to form a rigid proximal end and a flexible distal end for a balance between the pushing force and the vascular compliance. However, their gradient changes are based on a preset static material distribution (such as tapered wall thickness or sparse metal mesh), making it difficult to dynamically adjust the stiffness of specific sections according to the actual degree of vascular tortuosity encountered during surgery. Moreover, the decreasing stiffness design (such as a flexible distal end) reduces the risk of damage to blood vessels due to the bending of the catheter, but at the cost of the radial compression resistance. Especially in a tortuous vessel, the low-stiffness segment at the distal end is prone to collapse and folding of the catheter due to the compression of the vascular wall. In contrast, the high-stiffness segment at the proximal end can resist the compression, but may scratch the vascular endothelium due to excessive stiffness when the catheter passes through a curved segment.

### SUMMARY

The purpose of the present application is to provide an anti-bending intracranial double-tube guide catheter to solve one of the technical problems mentioned above in the prior art.

Specifically, the present application is realized by means of the following technical solution:
an anti-bending intracranial double-tube guide catheter, including a catheter body formed by an outer catheter and an inner catheter inserted in the outer catheter, and at least two anti-bending sections are arranged at an interval along the axial direction of the catheter body, with an anti-bending support mechanism provided in each of the anti-bending sections;
the anti-bending support mechanism includes an annular cavity and a plurality of memory alloy spiral wires; the annular cavity is arranged in a wall layer of the outer catheter; the plurality of memory alloy spiral wires are circumferentially arranged in the annular cavity; temperature control tubes are provided in the annular cavity and in intervals between the plurality of memory alloy spiral wires; and each of the temperature control tubes is S-shaped in the annular cavity, and is connected to the outside by means of a circulation pipe penetrating the wall layer of the outer catheter; and
a temperature medium is injected into each of the temperature control tubes by means of the circulation pipe, to activate a shape memory effect of the memory alloy spiral wires by temperature.

According to the technical solution described above, with the anti-bending support mechanisms provided in the catheter body, the temperature-sensitive characteristics and phase transition behavior of the memory alloy spiral wires are ingeniously utilized to realize dynamic adjustment to the bending stiffness of the catheter body; the core of the technical solution is that after a corresponding temperature medium is injected into the temperature control tubes in the annular cavity of each of the anti-bending support mechanisms by means of the circulation pipes, the temperature medium flows in the temperature control tubes, to conduct heat to the memory alloy spiral wires inside; when each of the memory alloy spiral wires reaches an appropriate temperature, a shape memory effect will occur and hardening of each of the memory alloy spiral wires will be triggered, namely a transition from the martensite phase to the austenite phase, so that each of the memory alloy spiral wires restores a preset high-stiffness geometry, thereby enhancing the bending stiffness of the corresponding section; and in addition, the circumferential arrangement of the memory alloy spiral wires can further improve the radial compression resistance of the outer catheter of the catheter body to resist vascular compression or external loads, and make the stress load on the outside of the catheter body be evenly distributed, thereby preventing the catheter body from collapsing due to compression; and
further, the anti-bending support mechanism further includes an elastic support layer attached to an outer wall of the inner catheter, and the elastic support layer is correspondingly arranged and adapted to the annular cavity.

The introduction of the elastic support layer into the technical solution further enhances the dynamic stiffness coordination and control capability of the inner catheter. As the elastic support layer is attached to the outer wall of the inner catheter and aligned with the annular cavity, when the memory alloy spiral wires are not activated (namely in the martensitic state at low temperature), the elastic support layer provides basic bending support for the inner catheter with its inherent elastic modulus to prevent the inner catheter from collapsing under low stiffness conditions. When the memory alloy spiral wires restore their stiffness in the austenite state, the outer catheter can form a composite support structure with the elastic support layer of the inner catheter in the radial direction. This structure not only buffers the sudden stiffness change of the outer catheter due to the memory alloy spiral wires by means of its own elastic deformation to prevent squeezing the inner catheter, but also strengthens the uniform radial support of the circumferential arrangement of the memory alloy spiral wires with its own resilience force, thereby enhancing the bending stiffness of the inner catheter and the outer catheter by means of their coordination.

Specifically, the annular cavity is provided with a plurality of mounting grooves for adaptively accommodating the memory alloy spiral wires; two ends of each of the memory alloy spiral wires are anchored to the corresponding mounting groove by means of limit convex points, and a middle section of each of the memory alloy spiral wires is a free deformation zone; the plurality of mounting grooves force the annular cavity to form arc-shaped bulges that bulge out toward the side of the inner catheter; and buffer positioning grooves are formed between adjacent arc-shaped bulges; and
a plurality of cushion pad layers adapted to the buffer positioning grooves are provided outside the elastic support layer.

According to the technical solution described above, when an external temperature medium enters the temperature control tubes in the annular cavity, a shape memory effect will occur in the memory alloy spiral wires, and a phase transition will be caused. For each of the memory alloy spiral wires, with the two ends constrained, the middle section, as a free deformation zone, can return to a preset high-stiffness geometry, which is specifically that the spiral radius of the middle section of each of the memory alloy spiral wires decreases and shrinks radially, to increase the axial and radial stiffness of each of the memory alloy spiral wires, thereby enhancing the bending resistance and radial compression resistance of the outer catheter, and significantly enhancing the bending stiffness of the outer catheter in the target section; and
moreover, with the arc-shaped bulges on the inner wall of the annular cavity, the buffer positioning grooves formed between adjacent arc-shaped bulges can form an engaged structure with the cushion pad layers of the elastic support layer on the outer wall of the inner catheter, so that when a bending force is applied on the outer catheter, the area with the arc-shaped bulges transmits the supporting force to the inner catheter by means of the cushion pad layers, and the deformation space of the buffer positioning grooves allows a certain radial compression of the elastic support layers to absorb the bending energy for a buffer effect, to prevent the inner catheter from being damaged by excessive extrusion due to its stiffness, thereby enhancing the bending stiffness of the entire catheter body.

Further, the buffer positioning grooves and the cushion pad layers also facilitate the mounting and positioning of the inner catheter and the outer catheter to a certain extent, thereby avoiding relative movement of the inner catheter and the outer catheter when they bend, which otherwise would cause inconvenience to the operation of interventional surgery.

Preferably, the two anti-bending sections are located at a proximal end and a distal end of the catheter body respectively, and when the shape memory effect occurs, the temperature of the memory alloy spiral wires in the annular cavity of each of the two anti-bending sections decreases along the direction in which the temperature medium flows in the circulation pipe.

Further, the memory alloy spiral wires of the two anti-bending sections are designed with different temperature thresholds where the shape memory effect occurs and a phase transition is caused. Specifically, the temperature where the shape memory effect occurs in the memory alloy spiral wires in the anti-bending section at the distal end of the catheter body is lower than that where the shape memory effect occurs in the memory alloy spiral wires in the anti-bending section at the proximal end of the catheter body.

It should be noted that since the anti-bending section at the proximal end and the anti-bending section at the distal end are designed with different memory alloy spiral wires with different temperature thresholds for phase transition, it is possible to inject temperature media of different temperatures to correspondingly adjust the memory alloy spiral wires at the proximal and distal ends of the catheter body respectively, thereby achieving differentiated stiffness adjustment to the anti-bending sections of the catheter body for different parts of intracranial vessels.

In addition, as a supplement, the distal end of the catheter body serves as an intervention operating end, and directly contacts the delicate and fragile endothelium of the vessel at the distal end. If the phase transition temperature is too high, the temperature medium may cause thermal damage to the vascular wall. Therefore, for the technical solution, the design that the temperature threshold for the memory alloy spiral wires at the distal end of the catheter body is lower than the temperature threshold for the memory alloy spiral wires at the proximal end of the catheter body can avoid thermal damage to the vascular wall to a certain extent. When the guide catheter is in operation, the proximal end of the catheter body is located in a larger vascular segment such as the internal carotid artery, where the vascular wall is thicker than that where the distal end is. Therefore, a higher temperature can activate the shape memory effect of the memory alloy spiral wires at the proximal end of the catheter body for phase transition, thereby enhancing the bending stiffness of the catheter body for supporting and pushing.

Specifically, the circulation pipe includes a first pipe, a second pipe, and a return pipe, and a medium outflow end of the first pipe is connected to an inlet of each of the temperature control tubes in the annular cavity at the proximal end of the catheter body;
the second pipe is located between the two anti-bending sections, and a medium outflow end of the second pipe is connected to an inlet of each of the temperature control tubes in the annular cavity at the distal end of the catheter body;
an outlet of each of the temperature control tubes in the annular cavity at the proximal end of the catheter body is connected to both a medium inflow end of the second pipe and a first medium inflow end of the return pipe, a second medium inflow end of the return pipe is connected to an outlet of each of the temperature control tubes in the annular cavity at the distal end of the catheter body, and a medium outflow end of the return pipe is connected to the outside; and
a regulating valve is provided at the connection between the medium outflow end of the second pipe and the inlet of each of the temperature control tubes in the annular cavity at the distal end of the catheter body, and an on-off assembly that matches the activation temperature of the memory alloy spiral wires in the annular cavity at the proximal end of the catheter body is provided in the regulating valve.

In other words, the on-off assembly is configured to be able to block the second pipe when the injected temperature medium reaches a temperature where a shape memory effect occurs in the memory alloy spiral wires in the annular cavity at the proximal end of the catheter body, thereby preventing the temperature medium from entering the annular cavity at the distal end of the catheter body.

The technical solution described above realizes independent temperature control and coordinated stiffness adjustment to the anti-bending sections at the proximal and distal ends of the catheter body, thereby enhancing the operational accuracy and safety of the catheter body in the complex intracranial vascular environment. Specifically, the design of the first pipe, the second pipe, and the return pipe, combined with the path control by the on-off assembly, ensures that a temperature medium of a corresponding temperature can flow to the annular cavity at the proximal or distal end of the catheter body in a targeted manner during specific operation, thereby realizing targeted activation of the memory alloy spiral wires at the proximal and distal ends of the catheter body, to ensure that the memory alloy spiral wires at the distal end of the catheter body are only activated by a low-temperature medium of 38°C at a position where a fragile vascular segment (such as the segment of the anterior cerebral artery) is, and the memory alloy spiral wires at the proximal end of the catheter body are activated only by a high-temperature medium of 40°C at a position where a high-resistance vascular segment (such as the petrous segment of the internal carotid artery) is. In this way, the anti-bending sections at the proximal and distal ends of the catheter body are designed with different temperature thresholds and temperature medium conveying paths that can be controlled independently, so that the bending stiffness of the proximal and distal ends of the catheter body can be adjusted independently.

Preferably, the on-off assembly includes a ball valve cavity, and a memory alloy ball valve core is adaptively provided in the ball valve cavity, and the temperature of the memory alloy ball valve core where the shape memory effect occurs is consistent with the temperature where the shape memory effect occurs in the memory alloy spiral wires in the anti-bending section at the proximal end of the catheter body. When the temperature reaches a value for the phase transition of the memory alloy spiral wires in the anti-bending section at the proximal end of the catheter body, the shape memory effect will occur in the shape memory alloy ball valve core, which will expand to fit tightly with the spherical cavity to block the second pipe.

Further preferably, the wall layer of the outer catheter includes an inner lining layer, a heat insulation layer, and a softening compatible layer from the inside out; the annular cavity, the first pipe, and the second pipe are located in the inner lining layer; the return pipe is located in the heat insulation layer; and thickened heat insulation buffer zones are formed on the heat insulation layer at positions corresponding to the anti-bending sections.

Further, the inner lining layer is configured to mainly serve as a bearing layer of the annular cavity and the circulation pipe, and the heat insulation layer can isolate the annular cavity from the outside of the catheter body to prevent heat conduction through the catheter body, thereby isolating the heat exchange between the intracranial vessels and the annular cavity in the catheter body to avoid mutual heat influence. In addition, a heat insulation buffer zone is provided at a corresponding position of the anti-bending section to further isolate the heat conduction between the catheter body and tissues, to ensure that the memory alloy spiral wires are activated normally and only affected by the temperature medium. In addition, the softening compatible layer is arranged at the outermost layer of the outer catheter, so that it can ensure affinity with intracranial vessels during interventional operation, thereby avoiding damage to the intracranial vessels.

Furthermore, the inner catheter is longer than the outer catheter, the distal end of the inner catheter extends out of the port at the distal end of the outer catheter to form an extension section for interventional operation, and a developing ring is provided at the end of the extension section. This design is convenient for operators to accurately position the extension section of the inner catheter by means of the developing ring during catheter intervention surgery.

Further specifically, an annular fitting portion is formed between the port at the distal end of the outer catheter and the outer wall of the inner catheter, and an anti-friction coating is provided on the annular fitting portion. The cooperation of the annular fitting portion and the anti-friction coating ensures that when the high-temperature medium activates the anti-bending section at the distal end of the catheter body, the inner catheter can still smoothly move in the outer catheter, thereby ensuring the normal operation of the inner catheter.

Compared with the prior art, the present application has the following advantages and beneficial effects:
1. according to the present application, with the anti-bending support mechanisms provided in the catheter body, the temperature-sensitive characteristics and phase transition behavior of the memory alloy spiral wires are ingeniously utilized to realize dynamic adjustment to the bending stiffness of the catheter body; the core of the technical solution is that after a corresponding temperature medium is injected into the temperature control tubes in the annular cavity of each of the anti-bending support mechanisms by means of the circulation pipes, the temperature medium flows in the temperature control tubes, to conduct heat to the memory alloy spiral wires inside; when each of the memory alloy spiral wires reaches an appropriate temperature, a shape memory effect will occur and hardening of each of the memory alloy spiral wires will be triggered, namely a transition from the martensite phase to the austenite phase, so that each of the memory alloy spiral wires restores a preset high-stiffness geometry, thereby enhancing the bending stiffness of the corresponding section; and in addition, the circumferential arrangement of the memory alloy spiral wires can further improve the radial compression resistance of the outer catheter of the catheter body to resist vascular compression or external loads, and make the stress load on the outside of the catheter body be evenly distributed, thereby preventing the catheter body from collapsing due to compression;
2. the introduction of the elastic support layer into the present application further enhances the dynamic stiffness coordination and control capability of the inner catheter; as the elastic support layer is attached to the outer wall of the inner catheter and aligned with the annular cavity, when the memory alloy spiral wires are not activated (namely in the martensitic state at low temperature), the elastic support layer provides basic bending support for the inner catheter with its inherent elastic modulus to prevent the inner catheter from collapsing under low stiffness conditions; when the memory alloy spiral wires restore their stiffness in the austenite state, the outer catheter can form a composite support structure with the elastic support layer of the inner catheter in the radial direction; and this structure not only buffers the sudden stiffness change of the outer catheter due to the memory alloy spiral wires by means of its own elastic deformation to prevent squeezing the inner catheter, but also strengthens the uniform radial support of the circumferential arrangement of the memory alloy spiral wires with its own resilience force, thereby enhancing the bending stiffness of the inner catheter and the outer catheter by means of their coordination; and
3. according to the present application, with the arc-shaped bulges on the inner wall of the annular cavity, the buffer positioning grooves formed between adjacent arc-shaped bulges can form an engaged structure with the cushion pad layers of the elastic support layer on the outer wall of the inner catheter, so that when a bending force is applied on the outer catheter, the area with the arc-shaped bulges transmits the supporting force to the inner catheter by means of the cushion pad layers, and the deformation space of the buffer positioning grooves allows a certain radial compression of the elastic support layers to absorb the bending energy for a buffer effect, to prevent the inner catheter from being damaged by excessive extrusion due to its stiffness, thereby enhancing the bending stiffness of the entire catheter body.

### BRIEF DESCRIPTION OF THE DRAWINGS

The drawings illustrated herein are used to provide a further understanding of the embodiments of the present application and constitute a part of the present application. They are not intended to limit the embodiments of the present application. Among the drawings:
FIG. 1 is a schematic diagram of the overall structure of an anti-bending intracranial double-tube guide catheter according to the present application;
FIG. 2 is a schematic diagram of the internal structure of an anti-bending section according to the present application, to show its specific internal state;
FIG. 3 is a schematic diagram of the cross-sectional view of the structure of an anti-bending section according to the present application, to show the internal structure of an annular cavity;
FIG. 4 is a schematic diagram of the half section view of the structure of an outer catheter according to the present application, to show the structure of an annular cavity;
FIG. 5 is a schematic diagram of the structure of a memory alloy spiral wire according to the present application, where FIG. 5(a) is intended to show the martensite phase state of the memory alloy spiral wire, and FIG. 5(b) is intended to show the austenite phase state of the memory alloy spiral wire;
FIG. 6 is a schematic diagram of the flow direction of a circulation pipe according to the present application, specifically when a regulating valve is closed;
FIG. 7 is a schematic diagram of the flow direction of a circulation pipe according to the present application, specifically when the regulating valve is opened;
FIG. 8 is a schematic diagram of the internal structure of a regulating valve according to the present application, specifically when a memory alloy ball valve core is opened;
FIG. 9 is a schematic diagram of the internal structure of a regulating valve according to the present application, specifically when the memory alloy ball valve core is closed; and
FIG. 10 is a schematic diagram of a partially enlarged view of the structure of an annular fitting portion according to the present application.

Reference numerals: 1. catheter body; 11. outer catheter; 111. inner lining layer; 112. heat insulation layer; 113. softening compatible layer; 12. inner catheter; 13. annular fitting portion; 2. anti-bending section; 21. annular cavity; 22. memory alloy spiral wire; 23. temperature control tube; 24. elastic support layer; 25. arc-shaped bulge; 26. cushion pad layer; 271. first pipe; 272. second pipe; 273. return pipe; 28. regulating valve; 281. ball valve cavity; 282. memory alloy ball valve core; 3. developing ring; 4. thermochromic ring; 5. joint; 6. Luer mount.

### DETAILED DESCRIPTION

In order to make the objectives, technical solution, and advantages of the present application more clear, the present application will be described in further detail below with reference to an embodiment and drawings. The illustrative embodiment method of the present application and its description are intended to explain the present application but not intended to limit the present application. It should be noted that the present application is already in the actual research and development stage.

### Embodiment

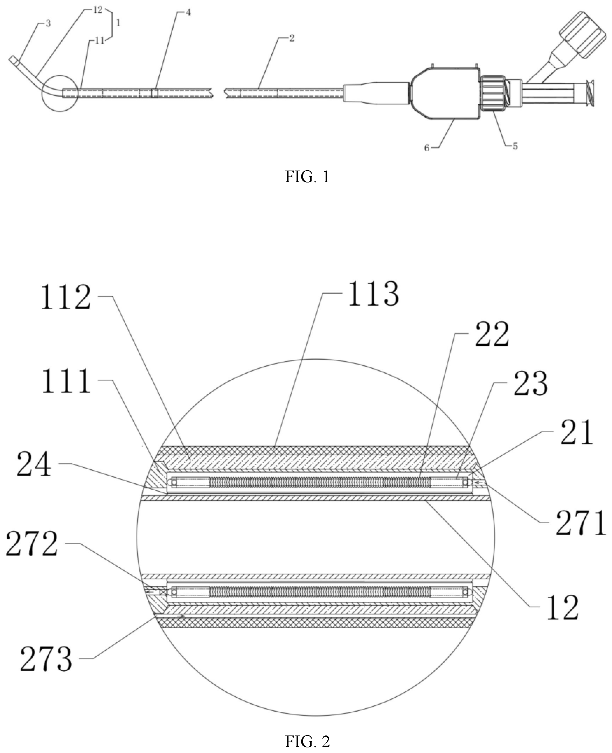

Referring to FIG. 1 to FIG. 10, this embodiment discloses an anti-bending intracranial double-tube guide catheter, including a catheter body 1 formed by an outer catheter 11 and an inner catheter 12 inserted in the outer catheter 11, and at least two anti-bending sections 2 are arranged at an interval along the axial direction of the catheter body 1, with an anti-bending support mechanism provided in each of the anti-bending sections 2;
the anti-bending support mechanism includes an annular cavity 21 and a plurality of memory alloy spiral wires 22; the annular cavity 21 is arranged in a wall layer of the outer catheter 11, the plurality of memory alloy spiral wires 22 are circumferentially arranged in the annular cavity 21, and temperature control tubes 23 are provided in the annular cavity 21 and in intervals between the plurality of memory alloy spiral wires 22; and each of the temperature control tubes 23 is S-shaped in the annular cavity 21, and is connected to the outside by means of a circulation pipe penetrating the wall layer of the outer catheter 11; and
a temperature medium is injected into each of the temperature control tubes 23 by means of the circulation pipe, to activate a shape memory effect of the memory alloy spiral wires 22 by temperature.

It should be noted that certain progress has been made on the conventional intracranial guide catheter technology so far, but there are still many shortcomings, because most catheters in the prior art are so designed that the stiffness decreases from the proximal end to the distal end. This method adapts to the changes in the diameter and curvature of intracranial vessels from a larger blood vessel at the proximal end to a smaller vessel at the distal end to a certain extent, which helps the advancement of the catheter in vessels. However, there are still certain defects in actual use. Since the bending stiffness of the entire catheter is predetermined and cannot be adjusted autonomously, it can hardly cope with the case when the vascular morphology differs greatly from the preset situation. For example, in some areas where the degree of vascular stenosis or tortuosity is far greater than expected, the low bending stiffness at the distal end makes it very easy for the catheter to bend excessively or even twist, so that the inner catheter 12 and the interventional instrument cannot pass smoothly, which seriously affects the surgical process. In the vascular segment at the proximal end that requires a large pushing force, if there is an abnormal increase in vascular wall resistance, the fixed stiffness cannot be enhanced according to actual needs, making it difficult for the operator to stably push the catheter to the target position, thereby greatly limiting the effectiveness of the catheter.

Therefore, the present application specifically proposes an anti-bending intracranial double-tube guide catheter disclosed in the embodiment described above, in which, with the anti-bending support mechanisms provided in the catheter body, the temperature-sensitive characteristics and phase transition behavior of the memory alloy spiral wires 22 are ingeniously utilized to realize dynamic adjustment to the bending stiffness of the catheter body 1; the core of the technical solution is that after a corresponding temperature medium is injected into the temperature control tubes 23 in the annular cavity 21 of each of the anti-bending support mechanisms by means of the circulation pipes, the temperature medium flows in the temperature control tubes 23, to conduct heat to the memory alloy spiral wires 22 inside; when each of the memory alloy spiral wires 22 reaches an appropriate phase transition temperature, the shape memory effect will occur and hardening of each of the memory alloy spiral wires 22 will be triggered, namely a transition from the martensite phase to the austenite phase, so that each of the memory alloy spiral wires 22 restores a preset high-stiffness geometry, thereby enhancing and adjusting the bending stiffness of the corresponding anti-bending section 2; and in addition, the circumferential arrangement of the memory alloy spiral wires 22 can further improve the radial compression resistance of the outer catheter 11 of the catheter body 1 to resist vascular compression or external loads, and make the stress load on the outside of the catheter body 1 be evenly distributed, thereby preventing the catheter body 1 from collapsing due to compression;

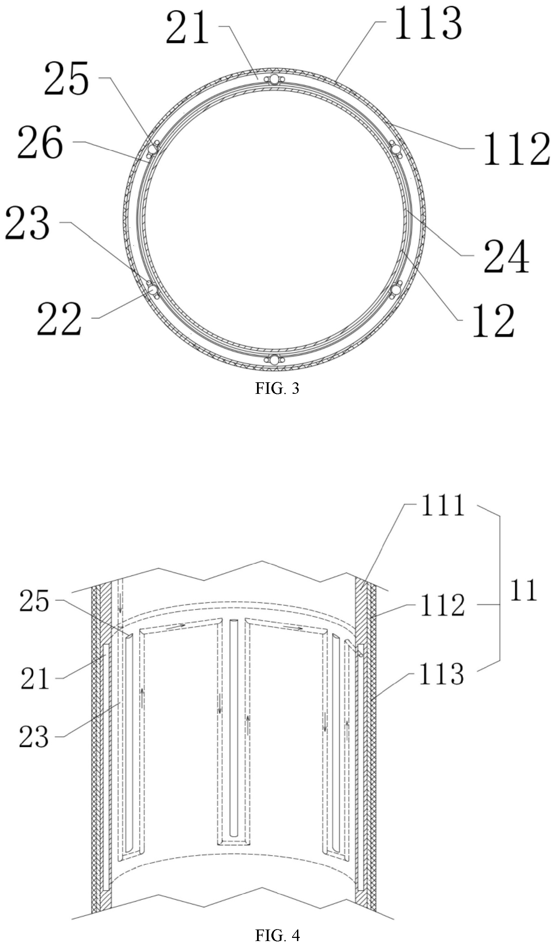

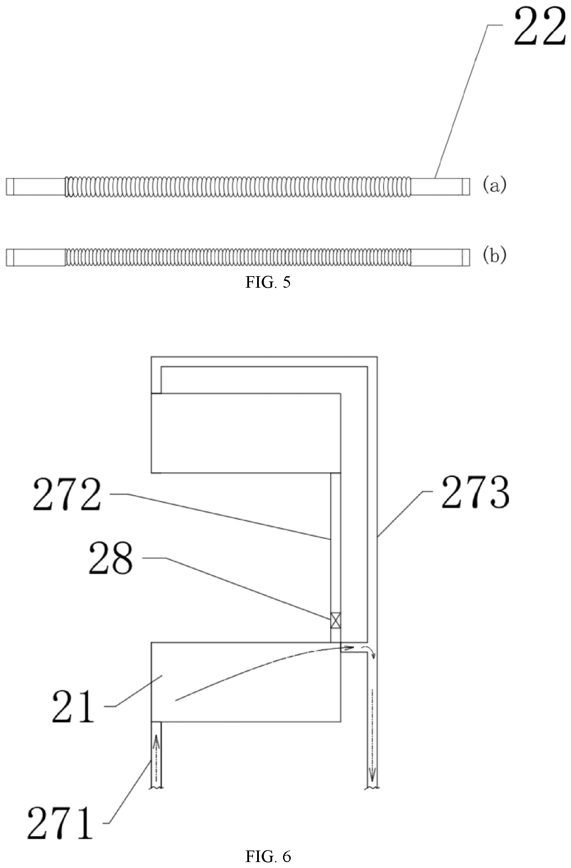

Further, according to the technical solution, when each of the memory alloy spiral wires 22 reaches an appropriate temperature, the shape memory effect occurs to make it restore to the initial high-stiffness shape, thereby adjusting the mechanical properties of the corresponding anti-bending section 2, to dynamically adjust the bending stiffness of the catheter body 1. Specifically, six to eight memory alloy spiral wires 22 are evenly distributed around the annular cavity 21. As shown in FIG. 3, six memory alloy spiral wires 22 are preferred in this embodiment. The memory alloy spiral wires 22 are made of Nitinol alloy, which has two crystal forms, namely martensite and austenite (as shown in FIG. 5). At low temperatures, the memory alloy spiral wires 22 are in the martensitic phase with good flexibility, and the crystal structure presents an easily deformable orthorhombic lattice, so that the memory alloy spiral wires 22 can bend naturally when the catheter body 1 moves in vessels for easy operation. When the temperature rises to a value higher than the phase transition temperature, the memory alloy spiral wires 22 absorb heat, and the lattice structure is reformed, transforming into the austenite phase of a regular face-centered cubic lattice. Then, each of the memory alloy spiral wires 22 returns to a preset high-stiffness spiral shape, significantly enhancing its ability to resist external bending. In this way, when different temperature media are injected into the temperature control tubes 23 by means of the circulation pipes, the memory alloy spiral wires 22 are accurately controlled to switch between the two forms, to dynamically adjust the bending stiffness of the catheter body 1.

As a further example, the memory alloy spiral wires 22 are made of Nitinol (NiTi), whose phase transition temperature can be precisely controlled by the ratio of alloy components. Specifically:
the phase transition temperature can be reduced by accurately increasing the nickel (Ni) content during preparation, and typical ratios are as follows:
the ratio for the memory alloy spiral wires 22 at the proximal end (which requires a higher temperature threshold) is that the nickel (Ni) content is from 50.6% to 50.8%, resulting in a phase transition temperature of 40°C;
the ratio for the memory alloy spiral wires 22 at the distal end (which requires a lower temperature threshold) is that the nickel (Ni) content is from 50.9% to 51.1%, resulting in a phase transition temperature of 38°C; and
for the addition of any trace element, adding 0.5% or less of tantalum (Ta) or copper (Cu) can improve the phase transition temperature stability and the cycle life.

The shape memory training process is as follows:
winding each of the memory alloy spiral wires 22 in the austenite state (high temperature) to the target spiral shape (that is, a high-stiffness geometric shape with a reduced spiral radius and a tight pitch), applying a constant stress of 50-100 MPa, cooling to the martensitic state, and then unloading the stress. After this process, each of the memory alloy spiral wires 22 will retain the relaxed form in the martensitic state (that is, the spiral radius is large and the pitch is loose). Then, repeating the thermomechanical cycle 3 to 5 times to form a stable two-way shape memory characteristic.

Preferably, when the temperature medium is injected into and flows in the temperature control tube 23, its function is not only to transfer heat to activate the shape memory effect of the memory alloy spiral wires 22 for phase transition, but also to form a stable static pressure inside the temperature control tube 23, thereby enhancing the bending stiffness of the corresponding anti-bending section 2.

In a specific implementation, the temperature control tube 23 is preferably made of graphite-based composite materials to significantly improve the efficiency and uniformity of heat conduction.

Further, referring to FIG. 3, the anti-bending support mechanism further includes an elastic support layer 24 attached to an outer wall of the inner catheter 12, and the elastic support layer 24 is correspondingly arranged and adapted to the annular cavity 21.

It should be understood that most double-lumen catheters in the prior art adopt independent reinforcement design, and there is no coordination mechanism to enhance the stiffness of the outer catheter 11 and the inner catheter 12, which affects the normal use of the catheter during surgery; in view of this, the introduction of the elastic support layer 24 into the embodiment method further enhances the dynamic stiffness coordination and control capability of the inner catheter 12. As the elastic support layer 24 is attached to the outer wall of the inner catheter 12 and aligned with the annular cavity 21, when the memory alloy spiral wires 22 are not activated (namely in the martensitic state), the elastic support layer 24 provides basic bending support for the inner catheter 12 with its inherent elastic modulus to prevent the inner catheter 12 from collapsing under low stiffness conditions. When the memory alloy spiral wires 22 restore their stiffness in the austenite state, the outer catheter 11 can form a composite support structure with the elastic support layer 24 of the inner catheter 12 in the radial direction. This structure not only buffers the sudden stiffness change of the outer catheter 11 due to the memory alloy spiral wires 22 by means of its own elastic deformation to prevent squeezing the inner catheter 12, but also strengthens the uniform radial support of the circumferential arrangement of the memory alloy spiral wires 22 with its own resilience force, thereby enhancing the bending stiffness of the inner catheter 12 and the outer catheter 11 by means of their coordination.

Specifically, as shown in FIG. 2 and FIG. 3, the annular cavity 21 is provided with a plurality of mounting grooves for adaptively accommodating the memory alloy spiral wires 22; two ends of each of the memory alloy spiral wires 22 are anchored to the corresponding mounting groove by means of limit convex points, and a middle section of each of the memory alloy spiral wires 22 is a free deformation zone; the plurality of mounting grooves force the annular cavity 21 to form arc-shaped bulges 25 that bulge out toward the side of the inner catheter 12; and buffer positioning grooves are formed between adjacent arc-shaped bulges 25; and
a plurality of cushion pad layers 26 adapted to the buffer positioning grooves are provided outside the elastic support layer 24, as shown in FIG. 3.

According to the technical solution described above, it should be noted that when an external temperature medium enters the temperature control tubes 23 in the annular cavity 21, a shape memory effect will occur in the memory alloy spiral wires 22, and a phase transition will be caused, and for each of the memory alloy spiral wires 22, with the two ends constrained, the middle section, as a free deformation zone, can return to a preset high-stiffness geometry, which is specifically that the spiral radius of the middle section of each of the memory alloy spiral wires 22 decreases and shrinks radially (in other words, it returns to a preset high-strength tight spiral shape to form a radial preload on the wall of the outer catheter 11), to increase the axial and radial stiffness of each of the memory alloy spiral wires 22, so that the memory alloy spiral wires 22 form a higher radial support force on the inner wall of the outer catheter 11, thereby enhancing the bending resistance and radial compression resistance of the outer catheter 11, and significantly enhancing the bending stiffness of the outer catheter 11 in the target section; and
moreover, with the arc-shaped bulges 25 on the inner wall of the annular cavity 21, the buffer positioning grooves formed between adjacent arc-shaped bulges 25 can form an engaged structure with the cushion pad layers of the elastic support layer 24 on the outer wall of the inner catheter 12, so that when a bending force is applied on the outer catheter 11, the area with the arc-shaped bulges 25 transmits the supporting force to the inner catheter 12 by means of the cushion pad layers 26, and the deformation space of the buffer positioning grooves allows a certain radial compression of the elastic support layers 24 to absorb the bending energy for a buffer effect, to prevent the inner catheter 12 from being damaged by excessive extrusion due to its stiffness, thereby enhancing the bending stiffness of the entire catheter body 1.

Specifically, the elastic support layer 24 is made of a silicone rubber-polyurethane blend with a thickness of 0.3 mm and an elastic modulus of 25-35 MPa. The cushion pad layers 26 on its surface are made of porous silica gel with a porosity of 30% and a compression resilience ratio of 95% or greater.

Further, the buffer positioning grooves and the cushion pad layers 26 provided also facilitate the mounting and positioning of the inner catheter 12 and the outer catheter 11 to a certain extent, thereby avoiding relative movement of the inner catheter 12 and the outer catheter 11 when they bend, which otherwise would cause inconvenience to the operation of interventional surgery.

Preferably, as shown in FIG. 1, the two anti-bending sections 2 are located at a proximal end and a distal end of the catheter body 1 respectively, and when the shape memory effect occurs, the temperature of the memory alloy spiral wires 22 in the annular cavity 21 of each of the two anti-bending sections 2 decreases along the direction in which the temperature medium flows in the circulation pipe.

Further, the two anti-bending sections are designed with different temperature thresholds where a shape memory effect occurs in the memory alloy spiral wires 22 and causes phase transition. Specifically, the temperature where a shape memory effect occurs in the memory alloy spiral wires 22 in the anti-bending section 2 at the distal end of the catheter body 1 is lower than that where a shape memory effect occurs in the memory alloy spiral wires 22 in the anti-bending section 2 at the proximal end of the catheter body 1.

It should be noted that since the anti-bending section 2 at the proximal end and the anti-bending section 2 at the distal end are designed with different temperature thresholds for the phase transition of the memory alloy spiral wires 22, it is possible to inject temperature media of different temperatures to correspondingly adjust the memory alloy spiral wires 22 at the proximal and distal ends of the catheter body 1 respectively, thereby achieving differentiated stiffness adjustment to the anti-bending sections 2 of the catheter body 1 for different parts of intracranial vessels.

For example, during interventional operation, the outer catheter 11 and the inner catheter 12 are rotatably connected to each other by means of a joint 5 at the end of the inner catheter 12 and a Luer mount 6 at the end of the outer catheter 11, and then further fixed together. Therefore, in the technical solution, the proximal end of the catheter body 1 is close to the Luer mount 6 and the joint 5, and is located at the first 1/3 of the total length of the catheter body 1 (for example, when the total length of the catheter is 1500 mm, the range of the proximal end is 100-500 mm). The distal end of the catheter body 1 enters the skull near the catheter body 1 and is located at the rear 1/3 of the total length of the catheter body 1 (for example, when the total length of the catheter is 1500 mm, the range of the distal end is 1000-1500 mm).

Exemplarily, the temperature threshold where the shape memory effect occurs in the memory alloy spiral wires 22 at the proximal end of the catheter body 1 is set to 40°C, to prevent them from being mistakenly activated by the normal temperature of human tissues during interventional operation, thereby ensuring that the shape memory effect is only triggered when it is necessary to adjust the bending stiffness. Moreover, this temperature threshold is lower than the high temperature (42°C) tolerated by human tissues during interventional operation to avoid thermal damage to intracranial vascular tissues;
the temperature threshold where the shape memory effect occurs in the memory alloy spiral wires 22 at the distal end of the catheter body 1 is set to 38°C, which is close to human body temperature and slightly higher, so that this temperature threshold can also prevent the memory alloy spiral wires 22 from being mistakenly activated by the normal temperature of human tissues, and there is a significant difference from the temperature threshold where the shape memory effect occurs in the memory alloy spiral wires 22 at the proximal end of the catheter body 1, which can avoid the mutual influence between the memory alloy spiral wires 22 at the two ends when the temperature media of the corresponding temperatures are injected; and
further, the distal end of the catheter body 1 serves as an intervention operating end, and directly contacts the delicate and fragile endothelium of the vessel at the distal end. If the phase transition temperature is too high, the temperature medium may cause thermal damage to the vascular wall. Therefore, for the technical solution, the design that the temperature threshold for the memory alloy spiral wires 22 at the distal end of the catheter body 1 is lower than the temperature threshold for the memory alloy spiral wires 22 at the proximal end of the catheter body 1 can avoid thermal damage to the vascular wall to a certain extent. When the guide catheter is in operation, the proximal end of the catheter body 1 is located in a larger vascular segment such as the internal carotid artery, where the vascular wall is thicker than that where the distal end is. Therefore, a higher temperature can activate the shape memory effect of the memory alloy spiral wires 22 at the proximal end of the catheter body 1 for phase transition, thereby enhancing the bending stiffness of the catheter body 1 for supporting and pushing.

Specifically, as shown in FIG. 2, the circulation pipe includes a first pipe 271, a second pipe 272, and a return pipe 273, and a medium outflow end of the first pipe 271 is connected to an inlet of each of the temperature control tubes 23 in the annular cavity 21 at the proximal end of the catheter body 1;
the second pipe 272 is located between the two anti-bending sections, and a medium outflow end of the second pipe 272 is connected to an inlet of each of the temperature control tubes 23 in the annular cavity 21 at the distal end of the catheter body 1;
an outlet of each of the temperature control tubes 23 in the annular cavity 21 at the proximal end of the catheter body 1 is connected to both a medium inflow end of the second pipe 272 and a first medium inflow end of the return pipe 273, a second medium inflow end of the return pipe 273 is connected to an outlet of each of the temperature control tubes 23 in the annular cavity 21 at the distal end of the catheter body 1, and a medium outflow end of the return pipe 273 is connected to the outside; and
a regulating valve 28 is provided at the connection between the medium outflow end of the second pipe 272 and the inlet of each of the temperature control tubes 23 in the annular cavity 21 at the distal end of the catheter body 1, and an on-off assembly that matches the activation temperature of the memory alloy spiral wires 22 in the annular cavity 21 at the proximal end of the catheter body 1 is provided in the regulating valve 28.

That is, the on-off assembly is configured to be opened in the initial state, and be able to block the second pipe 272 when the injected temperature medium reaches a temperature where a shape memory effect occurs in the memory alloy spiral wires 22 in the annular cavity 21 at the proximal end of the catheter body 1, thereby preventing the temperature medium from entering the annular cavity 21 at the distal end of the catheter body 1.

The implementation described above realizes independent temperature control and coordinated stiffness adjustment to the anti-bending sections 2 at the proximal and distal ends of the catheter body 1, thereby enhancing the operational accuracy and safety of the catheter body 1 in the complex intracranial vascular environment. Specifically, the design of the first pipe 271, the second pipe 272, and the return pipe 273, combined with the path control by the on-off assembly, ensures that a temperature medium of a corresponding temperature can flow to the annular cavity 21 at the proximal or distal end of the catheter body 1 in a targeted manner during specific operation, thereby realizing targeted activation of the memory alloy spiral wires 22 at the proximal and distal ends of the catheter body 1, to ensure that the memory alloy spiral wires 22 at the distal end of the catheter body 1 are only activated by a low-temperature medium of 38°C at a position where a fragile vascular segment (such as the segment of the anterior cerebral artery) is, and the memory alloy spiral wires 22 at the proximal end of the catheter body 1 are activated only by a high-temperature medium of 40°C at a position where a high-resistance blood vessel segment (such as the petrous segment of the internal carotid artery) is. In this way, the anti-bending sections 2 at the proximal and distal ends of the catheter body 1 are designed with different temperature thresholds and temperature medium conveying paths that can be controlled independently, so that the bending stiffness of the proximal and distal ends of the catheter body 1 can be adjusted independently.

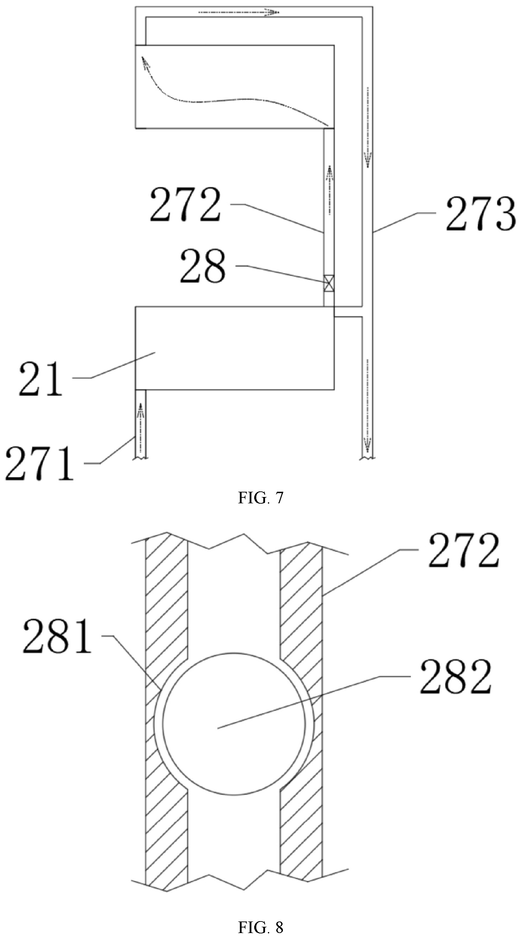

For example, as shown in FIG. 6, when it is necessary to enhance and adjust the bending stiffness of the anti-bending section 2 at the proximal end of the catheter body 1, a temperature medium of a corresponding temperature (such as sodium heparin saline at 40°C) is injected by means of the first pipe 271. When it enters the temperature control tube 23 in the annular cavity 21 at the proximal end of the catheter body 1, its heat is transferred to the surface of each of the memory alloy spiral wires 22 in the annular cavity 21, so that each of the memory alloy spiral wires 22 can reach the temperature threshold of phase transition, and then the shape memory effect occurs to trigger the phase transition (that is, each of the memory alloy spiral wires 22 returns to the preset high-stiffness geometry), thereby enhancing the bending stiffness of the anti-bending section 2 at the proximal end of the catheter body 1. Moreover, when the temperature medium flows to the regulating valve 28 of the second pipe 272, the on-off assembly of the valve also reaches the temperature threshold and blocks the second pipe 272, to prevent the temperature medium from flowing into the annular cavity 21 at the distal end of the catheter body 1 through the second pipe 272. The used temperature medium flows into a first medium inflow end of the return pipe 273 through the outlet of the temperature control tube 23 in the annular cavity 21, and returns to the external circulation; and
as shown in FIG. 7, when it is necessary to enhance and adjust the bending stiffness of the anti-bending section 2 at the distal end of the catheter body 1, a temperature medium of a corresponding temperature (such as sodium heparin saline at 38°C) can be injected by means of the first pipe 271, so that it first enters the temperature control tube 23 in the annular cavity 21 at the proximal end of the catheter body 1 through the first pipe 271 (then, since the temperature does not reach the activation temperature of the memory alloy spiral wires 22 there, the shape memory effect will not occur, that is, the memory alloy spiral wires 22 there remain flexible), and the on-off assembly will not block the second pipe 272. As a result, when the temperature medium enters the second pipe 272 and finally enters the temperature control tube 23 in the annular cavity 21 at the distal end, the temperature control tube 23 conducts heat to the surface of each of the memory alloy spiral wires 22 in the annular cavity 21 to trigger the shape memory effect of each of the memory alloy spiral wires 22 for phase transition (that is, it returns to the preset high-stiffness geometry), thereby enhancing the bending stiffness of the anti-bending section 2 at the distal end of the catheter body 1. The used temperature medium flows into a second medium inflow end of the return pipe 273 through the outlet of the temperature control tube 23 in the annular cavity 21 at the distal end of the catheter body 1, and returns to the external circulation.

In summary, the bending stiffness of the proximal and distal ends of the catheter body 1 can be accurately and independently adjusted to ensure the normal use of the catheter in complex intracranial vascular pathways. Moreover, with the different temperatures of the injected temperature media, the bending stiffness of the proximal or distal end of the catheter body 1 can be adjusted in different orders.

It can be understood that for the injection of temperature medium during interventional operation, an external temperature control pump is connected to the inlet interface of the first pipe 271 on the Luer mount 6 at the end of the outer catheter 11, and the medium output end of the return pipe 273 is connected to the external temperature control pump through the outflow interface on the Luer mount 6 at the end of the outer catheter 11. In this way, the temperature control pump is used to pump the temperature medium through the circulation pipe into the catheter body 1 for circulation, to dynamically adjust the bending stiffness of the proximal or distal end of the catheter body 1.

It should be further noted that in this embodiment, for the purpose of avoiding heat loss of the temperature medium during injection when it passes through the circulation pipe, a layer of nano-insulation film can be further coated on the inner wall of the first pipe 271 and the inner wall of the second pipe 272 of the circulation pipe, to avoid temperature drop of the temperature medium due to heat dissipation. The specific material of the nano-insulation film can only be selected specifically for actual implementation, and is not specifically described and further limited in the present application.

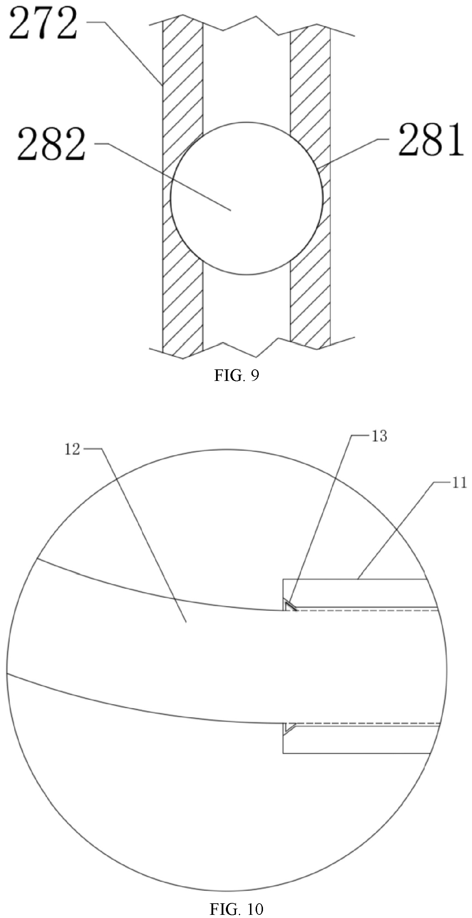

Preferably, as shown in FIG. 8 and FIG. 9, the on-off assembly includes a ball valve cavity 281, and a memory alloy ball valve core 282 is adaptively provided in the ball valve cavity 281, and the temperature of the memory alloy ball valve core 282 when the shape memory effect occurs is consistent with the temperature of the memory alloy spiral wires 22 in the anti-bending section 2 at the proximal end of the catheter body 1. When the temperature reaches a value for the phase transition of the memory alloy spiral wires 22 in the anti-bending section 2 at the proximal end of the catheter body 1, the shape memory effect will occur in the shape memory alloy ball valve core 282, which will expand to fit tightly with the spherical cavity to block the second pipe 272.

For example, the material of the memory alloy ball valve core 282 is consistent with the material of the memory alloy spiral wires 22 in the annular cavity 21 at the proximal end of the catheter body 1.

Further preferably, as shown in FIG. 2, FIG. 3, and FIG. 4, the wall layer of the outer catheter 11 includes an inner lining layer 111, a heat insulation layer 112, and a softening compatible layer 113 from the inside out; the annular cavity 21, the first pipe 271, and the second pipe 272 are located in the inner lining layer 111; the return pipe 273 is located in the heat insulation layer 112; and thickened heat insulation buffer zones are formed on the heat insulation layer 112 at positions corresponding to the anti-bending sections.

Further, the inner lining layer 111 is configured to mainly serve as a bearing layer of the annular cavity 21 and the circulation pipe and be preferably made of TPU, and the heat insulation layer 112 can isolate the annular cavity 21 from the outside of the catheter body 1 to prevent heat conduction through the catheter body 1, thereby isolating the heat exchange between the intracranial vessels and the annular cavity 21 in the catheter body 1 to avoid mutual heat influence. In addition, a heat insulation buffer zone is provided at a corresponding position of the anti-bending section to further isolate the heat conduction between the catheter body 1 and tissues, to ensure that the memory alloy spiral wires 22 are activated normally and only affected by the temperature medium. In addition, the softening compatible layer 113 is arranged at the outermost layer of the outer catheter 11, and the external surface is coated with a layer of hydrophilic material, so that it can ensure affinity with intracranial vessels during interventional operation, thereby avoiding damage to the intracranial vessels.

Further, the heat insulation layer 112 is preferably made of a transparent polyurethane material with good heat insulation performance (thermal conductivity ≤ 0.05 W/(m·K)), ensuring that the internal temperature of the catheter body 1 is always stable below 34°C when no temperature medium is injected (lower than the temperature threshold where the shape memory effect occurs in the memory alloy spiral wires 22 at the distal end, so as to avoid the influence of intracranial body temperature on the memory alloy spiral wire 22).

More preferably, as shown in FIG. 1, a thermochromic ring 4 is integrally formed on the inner wall of the heat insulation layer 112, with several thermochromic microcapsules arranged in the thermochromic ring 4. The thermochromic microcapsules are coated with crystal violet lactone (CVL) and an acid developer, with a color change temperature threshold precisely configured to be 38°C, which is consistent with the temperature where the shape memory effect occurs in the memory alloy spiral wires 22 at the distal end of the catheter body 1. As such, real-time visual monitoring of the temperature state of the distal end of the catheter during interventional operation can be achieved to eliminate the risk of overheating of the temperature medium or false activation of the memory alloy spiral wires 22, which will significantly improve the operation safety and convenience.

Further, spiral nano-grooves (50-200 nm deep and 80-150 nm wide, with a spacing of 5-10 µm between adjacent spiral nano-grooves, which are not shown in the figure) are provided on the outer surface of the softening compatible layer 113 along its axial direction. The three-dimensional structure of the nano-grooves can form micron-sized liquid storage units on the surface of the outer catheter 11, to contain body fluid or the corresponding physiological saline during interventional operation. As such, when the outer catheter 11 moves in a blood vessel, a continuously distributed lubricating liquid film is formed on the outer surface of the outer catheter 11, to further reduce the friction between the outer catheter 11 and the blood vessel and reduce the damage to the vascular wall.

Furthermore, the inner catheter 12 is longer than the outer catheter 11, the distal end of the inner catheter 12 extends out of the port at the distal end of the outer catheter 11 to form an extension section (which is 50-100 mm long) for interventional operation, and a developing ring 3 is provided at the end of the extension section. This design is convenient for operators to accurately position the extension section of the inner catheter 12 by means of the developing ring 3 during catheter intervention surgery.

Further specifically, as shown in FIG. 10, an annular fitting portion 13 is formed between the port at the distal end of the outer catheter 11 and the outer wall of the inner catheter 12; an anti-friction coating is provided on the annular fitting portion 13; and preferably, the anti-friction coating is a molybdenum disulfide layer with a thickness of 2-5 µm deposited by magnetron sputtering.

In a specific implementation, the cooperation of the annular fitting portion 13 and the anti-friction coating ensures that when the high-temperature medium activates the anti-bending section 2 at the distal end of the catheter body 1, the inner catheter 12 can still smoothly move in the outer catheter 11, thereby ensuring the normal operation of the inner catheter 12.

The specific implementation above further describes the objectives, technical solutions, and beneficial effects of the present application in detail. It should be understood that the above is only the specific embodiment method of the present application. The content about the specific type selected and related material process parameters is only used to explain the present application, but is not intended to limit the scope of protection of the present application. Any modification, equivalent replacement, or improvement within the spirit and principle of the present application shall be covered by the scope of protection of the present application.

It should be noted that the structures, proportions, and sizes shown in the attached figures of the specification are illustrative and only used to assist in explaining the content disclosed in the specification for understanding and reading by those skilled in the art, and are not conditions limiting the implementation of the present application. Therefore, they have no technical substantive significance. Any modifications to the structures, changes in proportional relationship, and adjustments in size shall fall within the scope of the technical content disclosed in the present application provided that they do not influence the effect and purpose of the present application. Meanwhile, the terms such as "upper", "lower", "left", "right", and "middle", cited in the specification, are also for the convenience of clear description only, rather than limiting the implementation scope of the present application, and the change or adjustment of the corresponding relation thereof is also deemed to fall in the implementation scope of the present application when the technical content is not substantially changed.

## Claims

1. An anti-bending intracranial double-tube guide catheter, comprising a catheter body (1) formed by an outer catheter (11) and an inner catheter (12) inserted in the outer catheter (11), wherein at least two anti-bending sections (2) are arranged at an interval along the axial direction of the catheter body (1), with an anti-bending support mechanism provided in each of the anti-bending sections (2);
the anti-bending support mechanism comprises an annular cavity (21) and a plurality of memory alloy spiral wires (22), wherein the annular cavity (21) is arranged in a wall layer of the outer catheter (11), the plurality of memory alloy spiral wires (22) are circumferentially arranged in the annular cavity (21), and temperature control tubes (23) are provided in the annular cavity (21) and in intervals between the plurality of memory alloy spiral wires (22), wherein each of the temperature control tubes (23) is S-shaped in the annular cavity (21), and is connected to the outside by means of a circulation pipe penetrating the wall layer of the outer catheter (11);
a temperature medium is injected into each of the temperature control tubes (23) by means of the circulation pipe, to activate the memory alloy spiral wires (22) by temperature, so that a shape memory effect occurs;
the two anti-bending sections (2) are located at a proximal end and a distal end of the catheter body (1) respectively, and when the shape memory effect occurs, the temperature of the memory alloy spiral wires (22) in the annular cavity (21) of each of the two anti-bending sections (2) decreases along the direction in which the temperature medium enters the circulation pipe;
the circulation pipe comprises a first pipe (271), a second pipe (272), and a return pipe (273), wherein a medium outflow end of the first pipe (271) is connected to an inlet of each of the temperature control tubes (23) in the annular cavity (21) at the proximal end of the catheter body (1);
the second pipe (272) is located between the two anti-bending sections, and a medium outflow end of the second pipe (272) is connected to an inlet of each of the temperature control tubes (23) in the annular cavity (21) at the distal end of the catheter body (1);
an outlet of each of the temperature control tubes (23) in the annular cavity (21) at the proximal end of the catheter body (1) is connected to both a medium inflow end of the second pipe (272) and a first medium inflow end of the return pipe (273), a second medium inflow end of the return pipe (273) is connected to an outlet of each of the temperature control tubes (23) in the annular cavity (21) at the distal end of the catheter body (1), and a medium outflow end of the return pipe (273) is connected to the outside;
a regulating valve (28) is provided at the connection between the medium outflow end of the second pipe (272) and the inlet of each of the temperature control tubes (23) in the annular cavity (21) at the distal end of the catheter body (1), and an on-off assembly that matches the activation temperature of the memory alloy spiral wires (22) in the annular cavity (21) at the proximal end of the catheter body (1) is provided in the regulating valve (28); and
the on-off assembly comprises a ball valve cavity (281), and a memory alloy ball valve core (282) is adaptively provided in the ball valve cavity (281), wherein the temperature of the memory alloy ball valve core (282) when the shape memory effect occurs is consistent with the temperature of the memory alloy spiral wires (22) in the anti-bending section at the proximal end of the catheter body (1).

2. The anti-bending intracranial double-tube guide catheter according to claim 1, wherein the anti-bending support mechanism further comprises an elastic support layer (24) attached to an outer wall of the inner catheter (12), and the elastic support layer (24) is correspondingly arranged and adapted to the annular cavity (21).

3. The anti-bending intracranial double-tube guide catheter according to claim 2, wherein the annular cavity (21) is provided with a plurality of mounting grooves for adaptively accommodating the memory alloy spiral wires (22), two ends of each of the memory alloy spiral wires (22) are anchored to the corresponding mounting groove by means of limit convex points, and a middle section of each of the memory alloy spiral wires (22) is a free deformation zone; the plurality of mounting grooves force the annular cavity (21) to form arc-shaped bulges (25) that bulge out toward the side of the inner catheter (12); and buffer positioning grooves are formed between adjacent arc-shaped bulges (25); and
a plurality of cushion pad layers (26) adapted to the buffer positioning grooves are provided outside the elastic support layer (24).

4. The anti-bending intracranial double-tube guide catheter according to claim 1, wherein the wall layer of the outer catheter (11) comprises an inner lining layer (111), a heat insulation layer (112), and a softening compatible layer (113) from the inside out, the annular cavity (21), the first pipe (271), and the second pipe (272) are located in the inner lining layer (111), the return pipe (273) is located in the heat insulation layer (112), and thickened heat insulation buffer zones are formed on the heat insulation layer (112) at positions corresponding to the anti-bending sections.

5. The anti-bending intracranial double-tube guide catheter according to claim 1, wherein the inner catheter (12) is longer than the outer catheter (11), the distal end of the inner catheter (12) extends out of the port at the distal end of the outer catheter (11) to form an extension section for interventional operation, and a developing ring (3) is provided at the end of the extension section.

6. The anti-bending intracranial double-tube guide catheter according to claim 5, wherein an annular fitting portion (13) is formed between the port at the distal end of the outer catheter (11) and the outer wall of the inner catheter (12), and an anti-friction coating is provided on the annular fitting portion (13).
